(19) Europäisches Patentamt
European Patent Office
Office européen des brevets

(11) **EP 3 106 139 A1**

(12) **EUROPÄISCHE PATENTANMELDUNG**

(43) Veröffentlichungstag:
**21.12.2016   Patentblatt 2016/51**

(51) Int Cl.:
*A61F 13/00* (2006.01)    *A61F 13/02* (2006.01)
*A61L 15/22* (2006.01)    *A61L 15/24* (2006.01)
*A61L 15/26* (2006.01)    *B01D 69/00* (2006.01)
*B01D 69/06* (2006.01)

(21) Anmeldenummer: **15172525.6**

(22) Anmeldetag: **17.06.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(71) Anmelder: **3M Innovative Properties Company
St. Paul, MN 55133-3427 (US)**

(72) Erfinder:
• **STRIPP, Walter**
**42389 Wuppertal (DE)**
• **SCHNEIDER, Frank**
**42349 Wuppertal (DE)**

(74) Vertreter: **CPW GmbH
Kasinostraße 19-21
42103 Wuppertal (DE)**

(54) **BRANDWUNDAUFLAGE MIT POLYAMIDMEMBRAN**

(57)    Brandwundauflage umfassend eine Polyamidmembran, dadurch gekennzeichnet, dass die Polyamidmembran einen Feuchtigkeitsgehalt von mindestens 300 Gew.%, relativ zum Gewicht der trockenen Polyamidmembran, hat.

Polyamidmembran zur Verwendung als Brandwundauflage, dadurch gekennzeichnet, dass die Polyamidmembran vor Aufbringen auf die Wunde einen Feuchtigkeitsgehalt von mindestens 300 Gew.%, relativ zum Gewicht der trockenen Polyamidmembran, hat.

**EP 3 106 139 A1**

**Beschreibung**

Die vorliegende Erfindung betrifft eine Brandwundauflage umfassend eine Polyamidmembran und eine Polyamidmembran zur Verwendung als Brandwundauflage.

Verbrennungen oder Verbrühungen der Haut bis einschließlich des 2. Grades sind mit Blasenbildung und starken Schmerzen verbunden. In solchen Fällen wirkt eine schnelle Kühlung unter beispielsweise fließendem Wasser schmerzlindernd und unterdrückt die Blasenbildung. Solche Kühlung schränkt den Aktionsradius des Verletzten ein, da er sich stets in der Nähe von fließendem Wasser aufhalten muss. Alternativ können Kühlpacks oder Kühlsalben verwendet werden. Ebenso sind Wundabdecktücher bekannt, die auch angefeuchtet als Wundverband bei Brandwunden anwendbar sind.

Kühlsalben hinterlassen Rückstände, verschmutzen die Kleidung oder können zu allergischen Reaktionen führen. Eisbeutel unterkühlen die Haut u.U. bis zum schmerzhaften Kühlen und führen bei größer flächigen Verbrennungen ggf. zu Kreislaufproblemen.

Die Aufgabe der vorliegenden Erfindung besteht darin, eine einfache Brandwundauflage zur Verfügung zu stellen, die schnellen Abtransport von durch Verbrennung oder Verbrühung in die Haut gebrachte Wärme ermöglicht und einen anhaltenden Kühleffekt bietet.

Diese Aufgabe wird gelöst durch eine Brandwundauflage eine Polyamidmembran umfassend, dadurch gekennzeichnet, dass die Polyamidmembran einen Feuchtigkeitsgehalt von mindestens 300 Gew.%, relativ zum Gewicht der trockenen Polyamidmembran, hat. Vorzugsweise hat die Polyamidmembran einen Feuchtigkeitsgehalt von mindestens 400 Gew.% oder 500 Gew.%, relativ zum Gewicht der trockenen Polyamidmembran. Der Feuchtigkeitsgehalt in der Polyamidmembran kann durch Einbringen von beispielsweise Wasser erreicht werden oder auch von wässrigen Lösungen. Geeignet sind beispielsweise Kälte-Salzmischungen wie $CaCl_2$ in Wasser oder andere kristallwasserenthaltene anorganische Salze in Wasser. Besonders geeignet zur Herstellung von Kältemischungen ist $CaCl_2*6H_2O$. Besonders bevorzugt wird Wasser verwendet.

Polyamidmembranen werden in vielfacher Weise eingesetzt, beispielsweise als Filtrationsmembranen. Es ist auch bekannt, Polyamidmembranen in der Wundbehandlung, beispielsweise als Wundauflage einzusetzen.

EP599589 beschreibt eine Wundauflage, die eine Filtrationsmembran und weitere Lagen umfasst. Die Filtrationsmembran kann aus Polyamid gefertigt sein. In diesem Fall dient die Filtrationsmembran dazu, Feuchtigkeit von der Wunde weg zu einer absorbierenden Lage zu leiten, also eine Wunddrainage.

In ähnlicher Weise fungiert eine Polyamidmembran in der US2005/226917. Auch hier dient die Polyamidmembran zum Abtransport von Flüssigkeit und Dampf von der Wunde zu den absorbierenden Lagen der Auflage, um die Wunde selbst trocken zu halten.

DE10014557 beschreibt eine Wundauflage in der eine poröse Membran (z.B. aus Polyamid) verwendet wird, um das Einwachsen von Zellen in die äußere, absorbierende Schicht der Wundauflage zu verhindern.

In der US3824998 wird ebenfalls die Verwendung einer Polyamidmembran als Brandwundenauflage beschrieben, allerdings wird die Membran so aufgebracht, dass sie keinen Wundkontakt mehr hat, sondern eine Art Ballon um das Wundareal formt, der eine Kontamination der Wunde verhindert.

Keine der vorgehend beschriebenen Schriften offenbart jedoch, dass eine Polyamidmembran zur Wundkühlung eingesetzt wird.

Die vorliegende Polyamidmembran kann verschiedene weitere Zusätze enthalten, beispielsweise schmerzstillende Wirkstofflösungen wie *N,N*-Dimethyl-3-[1-(2-pyridyl)-ethyl]-1*H*-inden-2-ethanamin, ein H1-Antihistaminika der ersten Generation mit ausgeprägten anticholinergen Wirkungen.

Weiterhin kann die Brandwundauflage mehrlagig sein. Bei mehrlagigen Auflagen befindet sich in der Anwendung die Polyamidmembran auf der der Wunde zugewandten Seite und ist in direktem Wundkontakt. Weitere Lagen können beispielsweise eine Außenlage und ggf. Mittellagen sein. Durch diese Lagen kann die Brandwundauflage weitere Funktionalitäten erhalten, beispielsweise eine erhöhte Wasseraufnahmekapazität, aber auch eine Tragestabilität an bewegten und unbewegten Körperstellen.

Die Brandwundauflage der vorliegenden Erfindung wird bevorzugt für die Kühlung von Brandwunden des ersten und zweiten Grades eingesetzt, d.h. Brandwunden bei denen die Epidermis und/oder Dermis betroffen ist. Die Wundauflage ist besonders geeignet zur Kühlung von geschlossenen Wunden, d.h. Wunden, aus denen kein Blut oder Wundflüssigkeit austritt.

Die Brandwundauflage der vorliegenden Erfindung umfasst erfindungsgemäß eine Polyamidmembran, im Besonderen eine poröse, semipermeable Polyamidmembran. Überraschenderweise wird bei Verwendung anderer Membranen, wie z.B. befeuchteter Membranen gefertigt aus Polyethylen (PE), Polypropylen (PP), Polyethylensulfon (PES) oder Polyvinylidenfluorid (PVDF) kein anhaltender Kühleffekt erreicht. Es wird vermutet, dass der anhaltende Kühleffekt auch durch die hydrophilen Eigenschaften des Polyamids begründet ist. Begünstigt durch einen geringen Kontaktwinkel mit Wasser oder wässrigen Flüssigkeiten infolge der hydrophilen Eigenschaften der Polyamidmembran, ist auch die innere Membranoberfläche der Polyamidmembran benetzt, d.h. die Membran ist mit Wasser oder wässrigen Flüssigkeiten gefüllt. Die Oberfläche der Wunde steht ständig in Kontakt mit der Membran und der darin enthaltenen Flüssigkeit, welche Wärmeenergie aufnimmt und durch Verdampfen wegleitet. Dadurch wird der Wundbereich anhaltend gekühlt.

Die Polyamidmembran der Wundauflage der Erfindung ist eine poröse, semipermeable Membran. Die Polyamidmembran hat vorzugsweise über mindestens 50% ihrer Wanddicke eine mittlere Porengröße von 0.1-10 $\mu$m, insbesondere im Bereich von 0.5 -5 $\mu$m. Hierdurch kann ein hinreichend hoher Kühleffekt erreicht werden. Der mittlere Porendurchmesser lässt sich nach der in der ASTM F 316-03 beschriebenen Methode mit Hilfe eines Capillary Flow Porometers (PMI[®]) ermitteln.

Weiterhin kann die Polyamidmembran in verschiedenen Dicken vorliegen. Bevorzugt sind Ausführungsformen, in denen die Polyamidmembran eine Dicke von mindestens 140 $\mu$m, noch bevorzugter von mindestens 200 $\mu$m hat. Zu dünne Polyamidmembranen können nicht den gewünschten anhaltenden Kühleffekt erzielen.

Die Volumenporosität der Membran liegt bevorzugt im Bereich von 60 bis 95% und besonders bevorzugt im Bereich von 80 bis 90%. Die Volumenporosität stellt das Verhältnis von Hohlraumvolumen zu Gesamtvolumen in der Polyamidmembran dar. Die Volumenporosität wird bestimmt durch Differenzwägung der trockenen und der anschließend in Silikonöl von bekannter Dichte getränkten Membran. Hierzu wird eine Probe von mindestens 0,5 g der zu untersuchenden Membran trocken eingewogen. Die Membranprobe wird anschließend in eine das Membranmaterial benetzende, jedoch nicht quellende Flüssigkeit für 24 Stunden eingelegt, so dass die Flüssigkeit in alle Poren eindringt. Eine geeignete Flüssigkeit ist ein Silikonöl mit einer Viskosität von 200 mPa s bei 25°C (Fa. Merck). Dies lässt sich visuell daran erkennen, dass die Membranprobe von einem opaken in einen glasigen, transparenten Zustand übergeht. Anschließend wird die Membranprobe aus der Flüssigkeit entnommen, an der Membranprobe anhaftende Flüssigkeit durch Zentrifugieren bei ca. 1800 g entfernt und die Masse der so vorbehandelten nassen, d.h. flüssigkeitsgefüllten Membranprobe bestimmt.

Die Volumenporosität in % wird nach folgender Formel bestimmt:

$$\text{Volumenporosität [\%]} = 100 \cdot \frac{(m_{nass} - m_{trocken})/\rho_{Flüss.}}{(m_{nass} - m_{trocken})/\rho_{Flüss.} + m_{trocken}/\rho_{Polymer}}$$

wobei bedeuten:

$m_{trocken}$ = Gewicht der trockenen Membranprobe
$m_{nass}$ = Gewicht der nassen, flüssigkeitsgefüllten Membranprobe
$\rho_{Flüss.}$ = Dichte der verwendeten Flüssigkeit
$\rho_{Polymer}$ = Dichte des Membranpolymers

Eine hohe Volumenporosität ist positiv, weil eine sehr poröse Membran mehr Flüssigkeit aufnehmen kann und dadurch ein länger anhaltender Kühleffekt erreicht wird.

In einer Ausführungsform hat die Oberfläche der Polyamidmembran der Erfindung einen Wasserkontaktwinkel von maximal 75°, vorzugsweise maximal 45°.

Der Wasserkontaktwinkel ist definiert als Winkel zwischen aufgebrachtem Flüssigkeits- (Wasser-) Tropfen zur Festkörper- (Membran-) Oberfläche.

Ein möglichst niedriger Wasserkontaktwinkel ist eine Voraussetzung für eine gut benetzbare, das heißt hydrophile Membran.

Verfahren zur Fertigung einer erfindungsgemäßen Polyamidmembran sind bekannt. EP1289636 beschreibt ein solches Verfahren.

Bevorzugt ist die Polyamidmembran aus Polyamid 6, Polyamid 6.6, Polyamid 6.1, Polyamid 4.6, Polyamid 11, Polyamid 12 und/oder Polyamid-Copolymere aufgebaut.

Die vorliegende Erfindung betrifft auch eine Polyamidmembran zur Verwendung als Brandwundauflage. Die erfindungsgemäße Polyamidmembran hat mindestens eine Feuchtigkeitsgehalt von 300 Gew.%, vorzugsweise mindestens 400 Gew.% relativ zum Gewicht der Polyamidmembran.

Bevorzugt wird die erfindungsgemäße Polyamidmembran zur Behandlung von Brandwunden, vor allem des ersten und zweiten Grades eingesetzt.

Für die genannte Verwendung sind poröse, semipermeable Polyamid-membranen im Besonderen geeignet.

Es ist möglich, die Brandwundauflage nach einiger Zeit der Verwendung erneut mit Wasser oder einer wässrigen Flüssigkeit zu befeuchten, sodass der Kühleffekt verlängert wird.

In einer Ausführungsform wird eine Polyamidmembran mit einer mittleren Porengröße von 0.1- 10 $\mu$m, vor-

zugsweise von 0.5 -5 μm verwendet.

Weiterhin sind Polyamidmembranen mit einer Stärke von mindestens 140 μm, vorzugsweise von mindestens 200 μm zur Verwendung als Wundauflage geeignet.

Bevorzugt wird eine Polyamidmembran als Brandwundauflage verwendet, die eine hohe Volumenporosität hat, im Besonderen einer Volumenporosität von mindestens 80%.

Die Oberfläche der verwendeten Polyamidmembran hat bevorzugt einen Wasserkontaktwinkel von maximal 75°, vorzugsweise maximal 45° hat.

Die Polyamidmembran zur Verwendung als Wundauflage enthält in einer bevorzugten Ausführungsform Polyamid 6, Polyamid 6.6, Polyamid 6.1, Polyamid 4.6, Polyamid 11, Polyamid 12 und/oder Polyamid-Copolymere.

**Patentansprüche**

1. Brandwundauflage umfassend eine Polyamidmembran, **dadurch gekennzeichnet, dass** die Polyamidmembran einen Feuchtigkeitsgehalt von mindestens 300 Gew.%, relativ zum Gewicht der trockenen Polyamidmembran, hat.

2. Brandwundauflage nach Anspruch 1, **dadurch gekennzeichnet, dass** die Polyamidmembran eine poröse, semipermeable Membran ist.

3. Brandwundauflage nach Anspruch 1 oder 2, **dadurch gekennzeichnet, dass** die Polyamidmembran über mindestens 50% ihrer Wanddicke eine mittlere Porengröße von 0.1- 10 μm, vorzugsweise von 0.5 -5 μm hat.

4. Brandwundauflage nach einem oder mehreren der vorangehenden Ansprüche, **dadurch gekennzeichnet, dass** die Polyamidmembran eine Dicke von mindestens 140 μm, vorzugsweise von mindestens 200 μm hat.

5. Brandwundauflage nach einem oder mehreren der vorangehenden Ansprüche, **dadurch gekennzeichnet, dass** die Polyamidmembran eine Volumenporosität von mindestens 80 % hat.

6. Brandwundauflage nach einem oder mehreren der vorangehenden Ansprüche, **dadurch gekennzeichnet, dass** die Oberfläche der Polyamidmembran einen Wasserkontaktwinkel von maximal 75°, vorzugsweise maximal 45° hat.

7. Brandwundauflage nach einem oder mehreren der vorangehenden Ansprüche, **dadurch gekennzeichnet, dass** die Polyamidmembran Polyamid 6, Polyamid 6.6, Polyamid 6.1, Polyamid 4.6, Polyamid 11, Polyamid 12 und/oder Polyamid-Copolymere enthält.

8. Polyamidmembran zur Verwendung als Brandwundauflage, **dadurch gekennzeichnet, dass** die Polyamidmembran vor Aufbringen auf die Wunde einen Feuchtigkeitsgehalt von mindestens 300 Gew.%, relativ zum Gewicht der trockenen Polyamidmembran, hat.

9. Polyamidmembran zur Verwendung nach Anspruch 8, **dadurch gekennzeichnet, dass** die Polyamidmembran eine poröse, semipermeable Membran ist.

10. Polyamidmembran zur Verwendung nach Anspruch 8 oder 9, **dadurch gekennzeichnet, dass** die Polyamidmembran über mindestens 50% ihrer Wanddicke eine mittlere Porengröße von 0.1- 10 μm, vorzugsweise von 0.5 -5 μm hat.

11. Polyamidmembran zur Verwendung nach einem der Ansprüche 8-10, **dadurch gekennzeichnet, dass** die Polyamidmembran eine Dicke von mindestens 140 μm, vorzugsweise von mindestens 200 μm hat.

12. Polyamidmembran zur Verwendung nach einem der Ansprüche 8-11, **dadurch gekennzeichnet, dass** die Polyamidmembran eine Volumenporosität von mindestens 80% hat.

13. Polyamidmembran zur Verwendung nach einem der Ansprüche 8-12, **dadurch gekennzeichnet, dass** die Oberfläche der Polyamidmembran einen Wasserkontaktwinkel von maximal 75°, vorzugsweise maximal 45° hat.

14. Polyamidmembran zur Verwendung nach einem der Ansprüche 8-13, **dadurch gekennzeichnet, dass** die Polyamidmembran Polyamid 6, Polyamid 6.6, Polyamid 6.1, Polyamid 4.6, Polyamid 11, Polyamid 12 und/oder Polyamid-Copolymere enthält.

Europäisches
Patentamt

European
Patent Office

Office européen
des brevets

**EUROPÄISCHER RECHERCHENBERICHT**

Nummer der Anmeldung

EP 15 17 2525

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 01/70153 A1 (LOHMANN THERAPIE SYST LTS [DE]; BERTHOLD ACHIM [DE]; MUELLER WALTER [D] 27. September 2001 (2001-09-27) * Seite 1, Zeilen 4-7,25-30 * * Seite 3, Zeilen 8-12 * * Seite 3, Zeile 26 - Seite 4, Zeile 17 * ----- | 1-14 | INV. A61F13/00 A61F13/02 A61L15/22 A61L15/24 A61L15/26 B01D69/00 B01D69/06 |
| X | GB 2 272 645 A (JOHNSON & JOHNSON MEDICAL [US]) 25. Mai 1994 (1994-05-25) * Seite 1, Zeilen 5-12 * * Seite 2, Zeilen 17-28 * * Seite 3, Zeilen 24-27 * * Seite 5, Zeilen 34-38 * ----- | 1-14 | |
| X | DE 10 2008 062824 A1 (HARTMANN PAUL AG [DE]) 1. Juli 2010 (2010-07-01) * Seite 2, Absatz 1 * * Seite 4, Absätze 20,23,24 * * Seite 5, Absatz 26 * * Ansprüche 1,4,6 * ----- | 1,4,7,8, 11,14 | |
| X | DE 10 2008 017746 A1 (BEIERSDORF AG [DE]) 8. Oktober 2009 (2009-10-08) * Seite 2, Absätze 1,6 * * Seite 3, Absatz 22 * * Seite 3, Absatz 25 - Seite 4, Absatz 28 * * Seite 4, Absatz 40 * ----- | 1,8 | |
| X | EP 2 752 176 A1 (BSN MEDICAL GMBH [DE]) 9. Juli 2014 (2014-07-09) * Seite 2, Absätze 1,5 * * Seite 3, Absätze 20,21 * * Seite 4, Absatz 26 * ----- -/-- | 1,8 | |

RECHERCHIERTE SACHGEBIETE (IPC)

A61F
A61L
B01D

Der vorliegende Recherchenbericht wurde für alle Patentansprüche erstellt

| Recherchenort | Abschlußdatum der Recherche | Prüfer |
|---|---|---|
| München | 7. Dezember 2015 | Beins, Ulrika |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes
    Dokument

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 15 17 2525

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | DE 10 2008 031182 A1 (HARTMANN PAUL AG [DE]) 7. Januar 2010 (2010-01-07) * Seite 2, Absätze 1,8,9 * * Seite 3, Absatz 13 * * Seite 5, Absatz 24-28 * ----- | 1-14 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 7. Dezember 2015 | Beins, Ulrika |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

................................................................

& : Mitglied der gleichen Patentfamilie, übereinstimmendes
Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 17 2525

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-12-2015

| Im Recherchenbericht angeführtes Patentdokument | Datum der Veröffentlichung | Mitglied(er) der Patentfamilie | Datum der Veröffentlichung |
|---|---|---|---|
| WO 0170153 A1 | 27-09-2001 | AT 360406 T | 15-05-2007 |
| | | DE 10014557 A1 | 04-10-2001 |
| | | EP 1272140 A1 | 08-01-2003 |
| | | JP 4601235 B2 | 22-12-2010 |
| | | JP 2003527200 A | 16-09-2003 |
| | | US 2003104039 A1 | 05-06-2003 |
| | | WO 0170153 A1 | 27-09-2001 |
| GB 2272645 A | 25-05-1994 | AT 176394 T | 15-02-1999 |
| | | AU 675669 B2 | 13-02-1997 |
| | | AU 5183493 A | 02-06-1994 |
| | | BR 9304790 A | 14-06-1994 |
| | | CA 2109672 A1 | 24-05-1994 |
| | | DE 69323398 D1 | 18-03-1999 |
| | | DE 69323398 T2 | 26-08-1999 |
| | | EP 0599589 A1 | 01-06-1994 |
| | | ES 2126635 T3 | 01-04-1999 |
| | | GB 2272645 A | 25-05-1994 |
| | | IN 179145 B | 06-09-1997 |
| | | JP 2875469 B2 | 31-03-1999 |
| | | JP H0716256 A | 20-01-1995 |
| | | US 5759570 A | 02-06-1998 |
| | | ZA 9308727 A | 22-05-1995 |
| DE 102008062824 A1 | 01-07-2010 | AU 2009331911 A1 | 21-07-2011 |
| | | CN 102264323 A | 30-11-2011 |
| | | DE 102008062824 A1 | 01-07-2010 |
| | | DK 2379033 T3 | 16-12-2013 |
| | | EP 2379033 A1 | 26-10-2011 |
| | | ES 2437613 T3 | 13-01-2014 |
| | | RU 2011129066 A | 27-01-2013 |
| | | US 2011313383 A1 | 22-12-2011 |
| | | WO 2010072395 A1 | 01-07-2010 |
| DE 102008017746 A1 | 08-10-2009 | DE 102008017746 A1 | 08-10-2009 |
| | | EP 2274020 A2 | 19-01-2011 |
| | | WO 2009124578 A2 | 15-10-2009 |
| EP 2752176 A1 | 09-07-2014 | EP 2752176 A1 | 09-07-2014 |
| | | US 2014194824 A1 | 10-07-2014 |
| DE 102008031182 A1 | 07-01-2010 | DE 102008031182 A1 | 07-01-2010 |
| | | EP 2310057 A2 | 20-04-2011 |
| | | US 2011117178 A1 | 19-05-2011 |
| | | WO 2010000450 A2 | 07-01-2010 |

EPO FORM P0461

Für nähere Einzelheiten zu diesem Anhang : siehe Amtsblatt des Europäischen Patentamts, Nr.12/82

**EP 3 106 139 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 599589 A
- US 2005226917 A
- DE 10014557
- US 3824998 A
- EP 1289636 A